# EUROPÄISCHE PATENTSCHRIFT

(11) **EP 3 824 794 B1**
(45) Veröffentlichungstag und Bekanntmachung des Hinweises auf die Patenterteilung: **28.12.2022**
(21) Anmeldenummer: 19210433.9
(22) Anmeldetag: 20.11.2019
(51) Int. Cl.: A61B 1/005, A61M 25/01, A61B 1/00

(54) **HANDGRIFF FÜR EIN ENDOSKOP, ENDOSKOP MIT EINEM SOLCHEN HANDGRIFF UND VERFAHREN ZUM ANORDNEN EINES SOLCHEN HANDGRIFFS AN EINEM ENDOSKOP**
HANDLE FOR ENDOSCOPE, ENDOSCOPE COMPRISING SUCH A HANDLE AND METHOD FOR ARRANGING SUCH A HANDLE ON AN ENDOSCOPE
POIGNÉE POUR UN ENDOSCOPE, ENDOSCOPE DOTÉ D'UNE TELLE POIGNÉE ET PROCÉDÉ D'AGENCEMENT D'UNE TELLE POIGNÉE SUR UN ENDOSCOPE

(43) Veröffentlichungstag der Anmeldung: 26.05.2021
(73) Patentinhaber: ATMOS MedizinTechnik GmbH & Co. KG, 79853 Lenzkirch (DE)
(72) Erfinder: Maik, Greiser, 79761 Waldshut-Tiengen (DE)
(74) Vertreter: Westphal, Mussgnug & Partner Patentanwälte mbB

(56) Entgegenhaltungen:
- WO-A1-2010/044862
- DE-A1- 3 943 403
- DE-A1- 19 839 188
- JP-A- 2004 358 012

## Beschreibung

Endoskopie ist ein insbesondere in der Medizin weit verbreitetes Diagnose- und/oder Behandlungsverfahren, bei dem ein Tubus, der insbesondere als starrer Schaft oder als flexibler Schlauch ausgeführt sein kann, eines Endoskops in ein Lumen eingeführt und/oder durch ein Lumen hindurchgeführt wird. Am distalen Ende des Tubus ist der Endoskopkopf angeordnet, der beispielsweise eine Beleuchtungsvorrichtung und eine Optik oder Kamera aufweisen kann, am proximalen Ende des Tubus befindet sich das Endoskopgehäuse, in dessen Innenraum beispielsweise Mittel zur Daten- und/oder Bildverarbeitung, eine elektronische Steuerung, Elemente der Stromversorgung und/oder weitere optische Komponenten, insbesondere ein Okular, angeordnet sind. Darüber hinaus finden sich am Endoskopgehäuse in vielen Fällen Bedienelemente für das Endoskop. Insbesondere ist bei sehr vielen Endoskopen ein Bedienelement zum Schwenken des Endoskopkopfes vorgesehen, das insbesondere als Stellhebel ausgeführt sein kann.

Während der gesamten Endoskopie muss der Benutzer des Endoskops im Regelfall das Endoskop am Endoskopgehäuse stabil unterstützen und durch geeignete, präzise und sanfte Bewegungen führen. Gerade bei längeren Untersuchungen oder Behandlungen kann sich dies als sehr ermüdend erweisen, weil die Ergonomie der Endoskopgehäuse marktüblicher Endoskope oft zu wünschen lässt, was dann negative Auswirkungen auf die Präzision, die der Benutzer erreicht, hat.

In Fällen, in denen das Endoskop längere Zeit im Wesentlichen ortsfest an einer bestimmten Stelle des Lumens bleibt, was insbesondere während endoskopischer Eingriffe der Fall ist, ist es bekannt, zur Vermeidung dieses Problems Haltevorrichtungen vorzusehen, die das Endoskopgehäuse eigenständig unterstützen, so dass sich der Bediener dann auf die Ausführung des Eingriffs konzentrieren kann.

Es ist ebenfalls bereits bekannt, einen ergonomisch günstigeren Handgriff nachträglich am Endoskopgehäuse zu befestigen, der dem Bediener das Halten des Endoskops erleichtert. Allerdings hat eine derartige Verschiebung der Griffposition den Nachteil, dass die Bedienelemente des Endoskops nicht mehr gut zu bedienen sind, weil sie herstellerseitig für eine andere Handposition des Benutzers optimiert angeordnet wurden.

Aus der WO 2010/044862 A1 und der DE 39 43 403 A1 sind jeweils Handgriffe für Endoskope bekannt, wobei WO 2010/044862 A1 die Merkmale des Oberbegriffs des Patentanspruchs 1 aufweist. Die Aufgabe der Erfindung besteht daher darin, einen Handgriff für ein Endoskop, ein Endoskop mit einem solchen Handgriff und ein Verfahren zum Anordnen eines solchen Handgriffs an einem Endoskop bereitzustellen, die gleichzeitig eine ergonomisch vorteilhafte Handhabung eines Endoskops und die Bedienung der Bedienelemente erlauben. Diese Aufgabe wird gelöst durch einen Handgriff für ein Endoskop mit den Merkmalen des Patentanspruchs 1, durch ein Endoskop mit den Merkmalen des Patentanspruchs 12 und durch ein Verfahren mit den Merkmalen des Patentanspruchs 13.

Vorteilhafte Weiterbildungen der Erfindung sind Gegenstand der jeweiligen abhängigen Ansprüche.

Der erfindungsgemäße Handgriff für ein Endoskop, ist für ein Endoskop ausgelegt, das ein Endoskopgehäuse und einen Tubus aufweist. Im Inneren des Endoskopgehäuses und des Tubus sind vorzugsweise die weiteren Komponenten des Endoskops in bekannter Weise angeordnet.

Wesentlich für einen bestimmungsgemäßen Einsatz des Handgriffs ist, dass an dem Endoskopgehäuse wenigstens ein Bedienelement zur Veränderung einer Einstellung des Endoskops vorhanden ist. Insbesondere kann dieses Bedienelement ein Hebel oder Bügel sein, mit dem ein am distalen Tubusende vorhandener Endoskopkopf geschwenkt werden kann, was beispielsweise über durch den Tubus geführte Bowdenzüge realisierbar ist.

Der erfindungsgemäße Handgriff selbst hat einen Aufnahmeabschnitt zur Aufnahme und Halterung des Endoskops, typischerweise von dessen Endoskopgehäuse, und einen mit dem Aufnahmeabschnitt verbundenen Griffabschnitt zum Halten des Endoskops durch seinen Benutzer.

Typischerweise kann der Aufnahmeabschnitt eine Öffnung oder eine nicht vollständig umrandete Ausnehmung aufweisen, die das Endoskopgehäuse ganz oder teilweise umgreift und deren Innenkontur vorzugsweise an das jeweilige Endoskopgehäuse angepasst ist. Das Endoskop kann in die Öffnung eingeschoben werden, wobei insbesondere bei konisch zulaufenden Endoskopgehäusen auch eine exakte Positionierung des Handgriffs am Endoskop erlaubt, bei mehrteiligen Aufnahmeabschnitten kann dieser aber auch geöffnet werden, um das Endoskop einzuführen und dann z.B. verschraubt oder verrastet werden oder um das Endoskop herumgebaut werden. Eine Fixierung des Endoskops im Aufnahmeabschnitt muss nicht zwingend unmittelbar durch den Aufnahmeabschnitt bewirkt werden, sondern kann beispielsweise auch mittels im oder am Aufnahmeabschnitt gelagerten Schrauben oder Klemmmitteln erreicht werden.

Erfindungswesentlich ist, dass der Handgriff ferner wenigstens einen Manipulator zur Bedienung des Bedienelements wenn der Handgriff an einem Endoskop angeordnet ist aufweist, wobei der Manipulator relativ zu dem Aufnahmeabschnitt und relativ zu dem Griffabschnitt beweglich ist.

Durch diese Maßnahme wird verhindert, dass der vorteilhafte Effekt, der durch die Bereitstellung einer ergonomisch vorteilhafteren Griffposition, die der Handgriff leistet, erzielt wird, ganz oder teilweise wieder dadurch zunichte gemacht wird, dass das Bedienelement des Endoskops für eine andere Griffposition optimiert angeordnet wurde und umgekehrt eine ergonomisch ideal an die durch den Handgriff vorgegebene Griffposition angepasste Möglichkeit zur Betätigung des Bedienelements geschaffen.

Vorzugsweise ist der Handgriff so ausgestaltet, dass er nachrüstbar ist. Dies ist gegeben, wenn er vom Endoskop getrennt werden kann und nicht einstückig an das Endoskop angeformt ist.

Vorteilhaft ist dabei eine pistolenartige Gesamtanordnung von Endoskop und Handgriff mit Manipulator, bei der der Lauf der Pistole durch das in den Aufnahmeabschnitt des Handgriffs aufgenommene Endoskop, der Griff der Pistole durch den Griffabschnitt des Handgriffs und der Abzug der Pistole durch den Manipulator beziehungsweise einen Teil des Manipulators gebildet wird.

Als besonders vorteilhaft haben sich Handgriffe erwiesen, bei denen der Aufnahmeabschnitt relativ zum Griffabschnitt abgewinkelt ist. Insbesondere bevorzugt ist dabei ein Abwinkeln, das so erfolgt, dass das vom Aufnahmeabschnitt abgewandte Ende des Griffabschnitts in Richtung auf das proximale Ende des bestimmungsgemäß im Halteabschnitt angeordneten Endoskops hin gekippt ist, besonders bevorzugt mit einem Winkel zwischen 5° und 45°.

Wenn der Handgriff Mittel zur Veränderung der Position des Aufnahmeabschnitts relativ zum Griffabschnitts aufweist, kann die Ergonomie individuell auf den Benutzer abgestimmt werden. Solche Mittel können bevorzugt dadurch realisiert sein, dass am Aufnahmeabschnitt ein Langloch oder eine Serie von Löchern bei unterschiedlichen Positionen vorgesehen ist, in bzw. an dem der Handgriff fixiert werden kann, beispielsweise durch Klemmen, Rasten oder Schrauben.

In einer vorteilhaften Variante der Erfindung ist der Aufnahmeabschnitt mindestens zweiteilig mit einem ersten Teil und einem zweiten Teil ausgeführt, wobei der Griffabschnitt und/oder der Manipulator werden zwischen dem ersten Teil und dem zweiten Teil gehalten. Dies schließt Konfigurationen ein, bei denen eine Öffnung zur Aufnahme des Endoskops nur in einem der Teile des Aufnahmeabschnitts vorhanden ist; andere Konfigurationen können aber auch vorsehen, dass die Öffnung durch das Zusammenwirken von mehreren Teilen des Aufnahmeabschnitts als Umrandung oder Begrenzung der Öffnung gebildet wird.

Besonders einfach ist dies realisierbar, wenn der Aufnahmeabschnitt einen ein- oder mehrteiligen Lagerabschnitt aufweist, an dem der Griffabschnitt und/oder der Manipulator gelagert sind. Dieser Lagerabschnitt bzw. eines der ihn bildenden Teile kann sich insbesondere von den Teilen des Aufnahmeabschnitts, die die Öffnung zur Aufnahme des Endoskops bilden, weg erstrecken und ist insoweit auch als mögliches Teil des Aufnahmeabschnitts interpretierbar.

Wenn der Manipulator eine Aufnahme zum zumindest abschnittsweisen Einführen des Bedienelements des Endoskops aufweist, kann auf einfache und sichere Weise die Verbindung zwischen Bedienelement und Manipulator hergestellt werden. Diese Aufnahme kann im einfachsten Fall durch ein Loch gebildet werden.

Bei einer weiteren vorteilhaften Ausgestaltung des Manipulators weist der Manipulator eine Aufnahme zum zumindest abschnittsweisen Einführen eines Fingers eines Anwenders des Endoskops auf, welche ebenfalls beispielsweise von einem Loch gebildet werden kann. Im Vergleich zu einer Anlagefläche ist der Vorteil einer solchen Aufnahme, dass sie es dem Benutzer erlaubt, ohne die Position des Fingers zu verändern sowohl Zug als auch Druck über den Manipulator auszuüben.

Um eine problemlose Funktion des Manipulators zu sichern ist es vorteilhaft, wenn der Manipulator zumindest abschnittsweise in einem Innenraum des Handgriffs verläuft.

Erfindungsgemäß weist der Manipulator einen ersten Abschnitt und einen zweiten Abschnitt auf, wobei der erste Abschnitt relativ zum zweiten Abschnitt um eine erste Drehachse herum drehbar ist und wobei der zweite Abschnitt relativ zum Aufnahmeabschnitt und/oder relativ zum Griffabschnitt um eine zweite Drehachse, die parallel zur ersten Drehachse verläuft, herum drehbar ist. Ein solcher Aufbau ermöglicht es insbesondere, dass der Manipulator auch ein Bedienelement, das beim Bedienen nicht nur linear verschoben, sondern entlang einer Kreisbahn bewegt wird, problemlos bedienen kann. Ferner kann durch Anpassung der Hebelverhältnisse des zweiten Abschnitts, bezogen auf die Lage der zweiten Drehachse im zweiten Abschnitt, einfach die gewünschte Feinheit der Einstellung angepasst werden. Gemäß einer anderen bevorzugten Weiterbildung des Handgriffs weist dieser einen Bildschirmaufnahmeabschnitt zur Aufnahme eines Bildschirms aufweist. Dies ermöglicht es, das Endoskop mit einem mobilen Bildschirm, der beispielsweise auch das Display eines Tablet-PCs oder Smartphones sein kann, auszurüsten, um die mit ihm gewonnenen Bilder unmittelbar anzuzeigen, zu speichern und/oder weiterzuleiten. Der Bildschirm kann optional mit seiner Stromversorgung auch das Endoskop mit Strom versorgen, was dann insgesamt zu einem besonders leichten und mobilen Endoskop führt.

Der Bildschirmaufnahmeabschnitt kann beispielsweise an der dem Griffabschnitt gegenüberliegenden Seite des Aufnahmeabschnitts oder seitlich am Aufnahmeabschnitt angeordnet sein. Vorteilhaft ist es, wenn er einen Bewegungsfreiheitsgrad bereitstellt, mit dem der Benutzer die Orientierung des Bildschirms, wenn er im Bildschirmaufnahmeabschnitt beziehungsweise eine Bildschirmaufnahme des Bildschirmaufnahmeabschnitts eingesetzt ist, noch individuell optimieren kann, beispielsweise durch einen Bildschirmaufnahmeabschnitt mit einem Dreh- oder Kugelgelenk.

Besonders bevorzugt ist dabei der Bildschirmaufnahmeabschnitt zur Aufnahme eines Bildschirms lösbar an dem Handgriff angeordnet. Dies kann beispielsweise durch magnetische Wechselwirkung, insbesondere zwischen einem an der entsprechenden Stelle des Aufnahmeabschnitts an oder in diesem angeordneten Magneten und einem am Bildschirmaufnahmeabschnitt angeordneten Magneten, und/oder durch mechanische Wechselwirkung, insbesondere Aufstecken, Aufrasten oder Aufschrauben geschehen. Die Verbindung wird vorzugsweise erst dann hergestellt, wenn das Endoskop bereits in den Aufnahmeabschnitt des Handgriffs eingeführt ist.

Das erfindungsgemäße Endoskop weist ein Endoskopgehäuse und einen Tubus auf, wobei an dem Endoskopgehäuse wenigstens ein Bedienelement zur Veränderung einer Einstellung des Endoskops vorhanden ist. Dieses Bedienelement kann insbesondere in einer besonders bevorzugten Variante ein Hebel, bevorzugt ein bügelförmiger Hebel sein, mit dem die Position des am distalen Ende des Tubus angeordneten Endoskopkopfs eingestellt werden kann.

Erfindungswesentlich ist, dass das Endoskop einen erfindungsgemäßen Handgriff, wie er vorstehend beschrieben wurde, aufweist.

Das erfindungsgemäße Verfahren zum Anordnen eines Handgriffs an einem Endoskop mit einem Endoskopgehäuse, wobei an dem Endoskopgehäuse wenigstens ein Bedienelement zur Veränderung einer Einstellung des Endoskops vorhanden ist, und mit einem Tubus, wird mit einem Handgriff ausgeführt, der einen Aufnahmeabschnitt zur Aufnahme und Halterung des Endoskops, einen mit dem Aufnahmeabschnitt -vorzugsweise aber nicht zwingend lösbar- verbundenen Griffabschnitt zum Halten des Endoskops und wenigstens einen Manipulator zur Bedienung des Bedienelements des Endoskops aufweist, wobei dieser Manipulator relativ zu dem Aufnahmeabschnitt und relativ zu dem Griffabschnitt beweglich ist. Das Verfahren weist insbesondere die Schritte
- Aufnehmen des Endoskops in einer Öffnung im Aufnahmeabschnitt des Handgriffs, und
- Verbinden des Bedienelements mit dem Manipulator auf.

Das Aufnehmen des Endoskops kann insbesondere dadurch erfolgen, dass das Endoskop in die Öffnung eingeschoben wird oder dass der Aufnahmeabschnitt derart mehrteilig ausgeführt ist, dass er um das Endoskop ganz oder teilweise herumgebaut werden kann oder beispielsweise auch dadurch, dass das Endoskops mit Schrauben oder Klemmmitteln am Aufnahmeabschnitt festgelegt wird.

Erfindungsgemäß ist der Manipulator während er mit dem Bedienelement verbunden wird vom Handgriff gelöst und wird nach dem Verbinden mit dem Bedienelement wieder oder erstmalig am Handgriff befestigt. Dies ermöglicht es, die Verbindung zwischen Aufnahmeabschnitt und Endoskop einerseits und zwischen Manipulator und Bedienelement andererseits unabhängig voneinander zu optimieren.

Dabei wird erfindungsgemäß ein Handgriff verwendet, der einen Manipulator hat, welcher einen ersten Abschnitt und einen zweiten Abschnitt aufweist, wobei der erste Abschnitt relativ zum zweiten Abschnitt um eine erste Drehachse herum drehbar ist und wobei der zweite Abschnitt relativ zum Aufnahmeabschnitt und/oder relativ zum Griffabschnitt um eine zweite Drehachse, die parallel zur ersten Drehachse verläuft, herum drehbar ist. Das

Bedienelement wird dann mit dem Manipulator verbunden, indem das Bedienelement in eine am ersten Abschnitt des Manipulators vorgesehene Aufnahmeöffnung eingeführt wird.

Der so mit dem Bedienelement verbundene Manipulator wird in einen Kanal im Aufnahmeabschnitt eingelegt und über die zweite Drehachse mit dem Aufnahmeabschnitt verbunden. Dies kann beispielsweise geschehen, indem der Manipulator auf die am Aufnahmeabschnitt bereits vorgesehene zweite Drehachse aufgesteckt wird oder dadurch, dass eine mit dem Manipulator verbundene zweite Drehachse in eine am Aufnahmeabschnitt vorgesesteckt wird oder dadurch, dass eine mit dem Manipulator verbundene zweite Drehachse in eine am Aufnahmeabschnitt vorgesehene Bohrung oder ein am Aufnahmeabschnit vorgesehenes Lager eingeführt wird.

Besonders bevorzugt ist dabei, wenn der Kanal nach dem Einlegen des mit dem Bedienelement verbundenen Manipulators und nach dem Verbinden über die zweite Drehachse mit dem Aufnahmeabschnitt mit einem Deckel, der z.B. aufgerastet oder aufgeschraubt werden kann verschlossen wird.

Eine vorteilhafte Variante des Verfahrens sieht vor, dass ein Handgriff verwendet wird, der einen Bildschirmaufnahmeabschnitt hat und dass ein Bildschirm in dem Bildschirmaufnahmeabschnitt angeordnet und zumindest eine Signalkommunikation mit dem Endoskop, beispielsweise durch ein Verbindungskabel zwischen Bildschirm und Endoskop, hergestellt wird, so dass auf dem Bildschirm, der beispielsweise das Display eines Smartphones oder eines Tablet-PCs, der in dem Bildschirmaufnahmeabschnitt angeordnet wird sein kann, die mit dem Endoskop gewonnene Bildinformation unmittelbar dargestellt wird. Auf diese Weise kann ein hochmobiles Endoskopsystem bereitgestellt werden, das noch weiter optimiert wird, wenn die Verbindung über das Verbindungskabel auch die Stromversorgung des Endoskops durch die Stromversorgung des Bildschirms, insbesondere durch einen Akku, ermöglicht.

In einer besonders bevorzugten Weiterbildung des Verfahrens wird ein Handgriff verwendet, der einen lösbaren Bildschirmaufnahmeabschnitt hat, wobei dieser lösbare Bildschirmaufnahmeabschnitt erst nach dem Aufnehmen des Endoskops (1) in einer Öffnung im Aufnahmeabschnitt des Handgriffs mit dem Handgriff verbunden wird. Insbesondere ist es weiter bevorzugt, wenn diese Verbindung auch erst nachdem die Verbindung zwischen Manipulator und Bedienelement hergestellt wird. Dadurch ist sichergestellt, dass die Bildschirmaufnahme, die oft ein relativ sperriges Bauteil ist, nicht bei der Durchführung der anderen Verfahrensschritte stört.

Die Erfindung wird nachfolgend anhand von Figuren, die ein Ausführungsbeispiel zeigen, näher erläutert. Es zeigt:
- Fig. 1:: Ein Ausführungsbeispiel eines Handgriffs beim Zusammenwirken mit einen Bedienelement eines Endoskops,
- Fig. 2:: eine Explosionsdarstellung der Anordung aus Handgriff und Bedienelement aus Figur 1,
- Fig. 3:: eine Seitenansicht eines Endoskops mit einem Handgriff wie in Figur 1 dargestellt in einer ersten Stellung des Bedienelements,
- Fig. 4:: eine Seitenansicht eines Endoskops mit einem Handgriff wie in Figur 1 dargestellt in einer zweiten Stellung des Bedienelements,
- Fig. 5:: eine Rückansicht einer ersten Variante des Handgriffs aus Figuren 1 und 2, und
- Fig. 6:: eine Rückansicht einer zweiten Variante des Handgriffs aus Figuren 1 und 2.

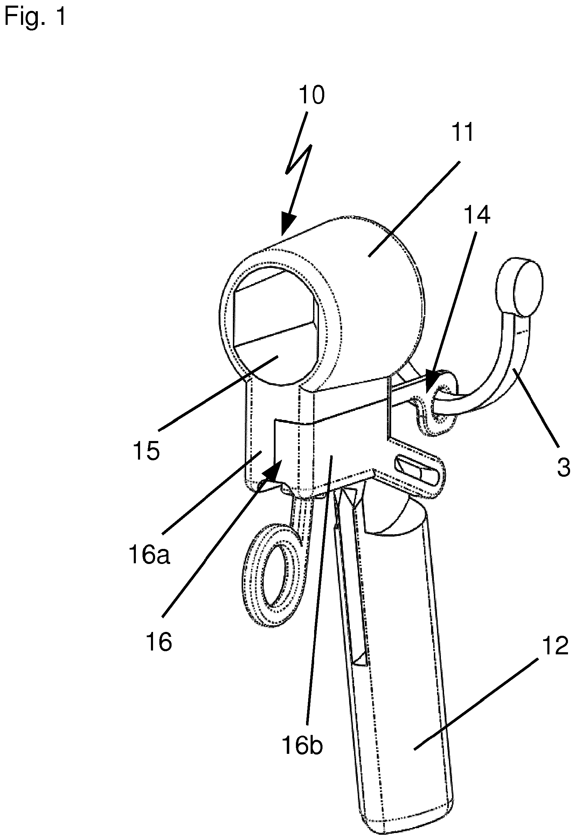

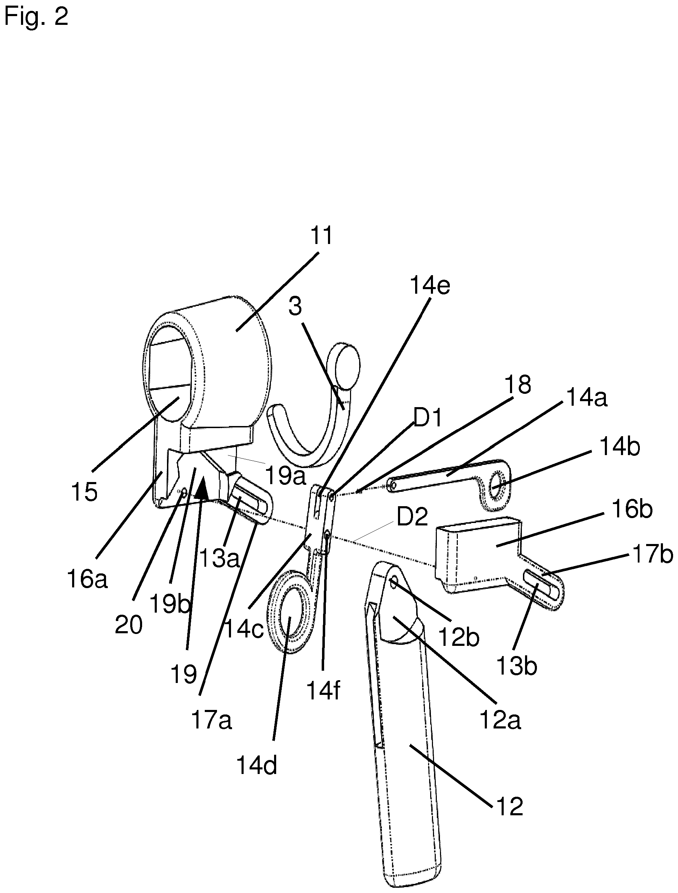

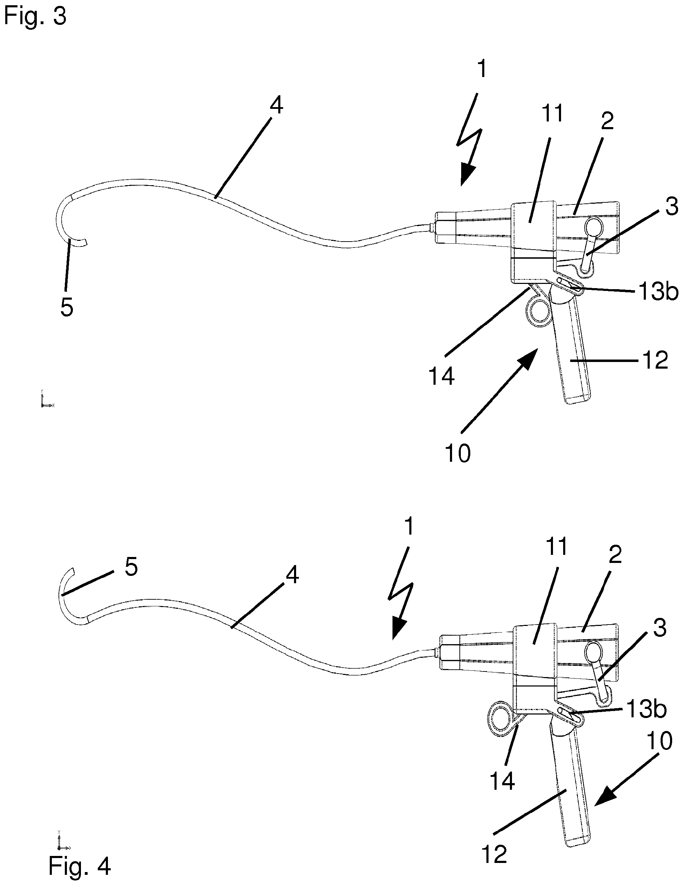

Figuren 1 und 2 zeigen einen Handgriff 10 für ein in diesen Figuren nicht dargestelltes, aber in Figuren 3 und 4 exemplarisch gezeigtes Endoskop 1 zusammen mit einem hier beispielhaft bügelförmig ausgeführten Bedienelement 3 des Endoskops 1, das über den Handgriff 10, genauer gesagt über einen an diesem angeordneten Manipulator 14, betätigbar ist.

Der Handgriff 10 weist einen Aufnahmeabschnitt 11 auf und hat ferner einen Griffabschnitt 12, dessen Position relativ zum Aufnahmeabschnitt 11 in Langlöchern 13a, 13b veränderbar ist, und zwar insbesondere auch hinsichtlich eines Kippwinkels relativ zum Aufnahmeabschnitt 11.

Der Manipulator 14 weist einen ersten Abschnitt 14a mit einer Aufnahme 14b zum zumindest abschnittsweisen Einführen des Bedienelements 3 auf und einen zweiten Abschnitt 14c mit einer Aufnahme 14d zum zumindest abschnittsweisen Einführen eines Fingers eines Anwenders des Endoskops 1 auf. Wie man in Figur 2 besonders gut erkennt, ist das dem ersten Abschnitt 14a zugewandte Teil des zweiten Abschnitts 14c mit einem Schlitz 14e zur Aufnahme des Endbereichs der Aufnahme 14b gegenüberliegenden Endbereichs des ersten Abschnitts 14a versehen und dort mit einem durch beide Abschnitte hindurch geführten Stift 18 um die durch den Stift 18 definierte erste Drehachse D1 herum drehbar fixiert, so dass der erste Abschnitt 14a des Manipulators 14 relativ zum zweiten Abschnitt 14b des Manipulators um die erste Drehachse D1 herum drehbar ist.

Der Aufnahmeabschnitt 11 umgibt -in diesem Fall mit einem einstückigen Rand, der aber ebenenfalls aus mehreren Teilen oder Segmenten zusammengesetzt sein kann- eine Öffnung 15 zur Aufnahme des Endoskops 1 ringförmig. Die Öffnung 15 ist dabei an die Form des Endoskopgehäuses 2 des Endoskops 1 angepasst. Wie man in der Darstellung der Figuren 3 und 4 erkennt, ist in diesem Beispiel ein in Richtung auf einen Tubus 4 hin konisch zulaufendes Endoskopgehäuse 2 dessen Außenkontur durch an zwei einander gegenüberliegenden Seiten abgeflachte Kreise beschrieben werden kann, so dass die Öffnung 15 als Folge ihrer Anpassung an das Endoskopgehäuse 3 eine konisch zulaufende Innenkontur hat, die durch an zwei einander gegenüberliegenden Seiten abgeflachte Kreise beschrieben werden kann. Dadurch ist zugleich definiert, wie weit das Endoskopgehäuse 3 in die Öffnung 15 einschiebbar ist.

Einstückig an den Aufnahmeabschnitt 11 angeformt und insoweit als ein Abschnitt des Aufnahmeabschnitts 11 interpretierbar ist ein erstes Teil 16a eines Lagerabschnitts 16, das mit einem deckelförmigen, vom ersten Teil 16a und damit auch vom Aufnahmeabschnitt 11 trennbaren zweiten Teil 16b des Lagerabschnitts 16 zusammenwirkt.

Der Lagerabschnitt 16 dient einerseits zur Lagerung des Griffabschnitts zwischen zwei Fortsätzen 17a des ersten Teils 16a des Lagerabschnitts 16 einerseits und 17b des zweiten Teils 16b des Lageabschnitts 16 andererseits, in denen die Langlöcher 13a, 13b angeordnet sind. Der Griffabschnitt 12 wird mit seinem Verbindungsbereich 12a so zwischen die Fortsätze 17a, 17b eingeführt, dass die Bohrung 12b mit den Langlöchern fluchtet, durch Verschieben und Kippen in die für den Benutzer ergonomisch günstigste Position gebracht und fixiert, was beispielsweise durch eine Klemmschraube oder ein Verklemmen, das bei einem Verrasten oder Verschrauben der Teile 16a, 16b des Lagerabschnitts 16 herbeigeführt werden kann, erfolgen kann.

Andererseits definiert der Lagerabschnitt 16 durch seinen ersten Teil 16a und den zweiten Teil 16b einen Kanal 19 zur Aufnahme von Teilbereichen des Manipulators 14. In einem ersten Abschnitt 19a des Kanals 19 ist ein Teilbereich des ersten Abschnitts 14a des Manipulators 14 aufgenommen. Dabei ist darauf hinzuweisen, dass die Breite b des Kanals 19 im Abschnitt 19a deutlich größer gewählt ist als die Breite des aufgenommenen Bereichs des Abschnitts 14a, so dass der Abschnitt 14a bei unterschiedlichen Positionen des Bedienelements 3 unter unterschiedlichen Winkeln durch den Kanal 19 verläuft. Dadurch wird es möglich, mit dem Manipulator 14 ein auf einer kreisförmigen Bahn bewegtes Bedienelement 3 zu kontrollieren.

In einem zweiten Abschnitt 19b des Kanals 19 ist ein Teilbereich des zweiten Abschnitts 14c des Manipulators 14 aufgenommen. Insbesondere weist der Lagerabschnitt 16 in diesem Bereich eine Bohrung 20 auf, in der der Manipulator 14-beispielsweise mittels einer am deckelartigen zweiten Teil 16b des Lagerabschnitts 16 angeformten, in den Figuren nicht sichtbaren Achse, die durch eine Bohrung 14f im zweiten Abschnitt 14c des Manipulators hindurch in die Bohrung 20 eingeführt wird, gelagert ist, so dass der zweite Abschnitt 14c des Manipulators 14 relativ zum Aufnahmeabschnitt 11 um eine zweite Drehachse D2, die parallel zur ersten Drehachse D1 verläuft, drehbar ist.

Der beschriebene Aufbau des Handgriffs 10 ermöglicht es insbesondere, separat voneinander die Verbindung zwischen Aufnahmeabschnitt 11 und Endoskop 1 bzw. Endoskopgehäuse 2 einerseits und Manipulator 14 und Betätigungselement 3 andererseits herzustellen und erst danach den Manipulator 14 mit dem Handgriff 10 zu verbinden, was beispielsweise bei bügelförmigen Betätigungselementen 3 von Endoskopen, deren Endoskopgehäuse keine rotationssymmetrische Geometrie hat und die deshalb im Aufnahmeabschnitt 11 nicht gedreht werden können, erforderlich ist. In Figur 3 und 4 erkennt man jeweils dasselbe Endoskop 1 mit Endoskopgehäuse 2, an dem das Bedienelement 3 angeordnet ist, mit Tubus 4 und mit Endoskopkopf 5. Das Bedienelement 3 dient dabei, wie man beim Vergleich der Figuren 3 und 4 unmittelbar erkennt, dazu, den Endoskopkopf 5 zu schwenken. Wenn das Bedienelement 3 an seinem in der Perspektive der Figuren 3 und 4 linken Anschlag steht, ist der Endoskopkopf 5 maximal nach unten gekrümmt; steht es an seinem in der Perspektive der Figuren 3 und 4 rechten Anschlag, ist der Endoskopfkopf 5 maximal nach oben gekrümmt.

Bei Betrachtung der Figuren 3 und 4 erkennt man ferner den wie oben anhand der Figuren 1 und 2 beschrieben aufgebauten Handgriff 10 mit Aufnahmeabschnitt 11, Griffabschnitt 12, dessen Position relativ zum Aufnahmeabschnitt 11 mittels des Langlochs 13 veränderbar ist und der vorzugsweise, wie in den Figuren 3 und 4, die eine reale Anwendungssituation darstellen, erkennbar ist, relativ zum Aufnahmeabschnitt 11 abgewinkelt verläuft. Ferner erkenn man am Handgriff 10 den Manipulator 14, welcher einen ersten Abschnitt 14a mit Aufnahme 14b zum zumindest abschnittsweisen Einführen des Bedienelements 3 aufweist und einen zweiten Abschnitt 14c mit Aufnahme 14d zum zumindest abschnittsweisen Einführen eines Fingers eines Anwenders des Endoskops 1 aufweist.

Bewegt der Benutzer des Endoskops 1 den zweiten Abschnitt 14c des Manipulators 14 so weit wie möglich an den Griffabschnitt 12 heran, wie es in Figur 3 gezeigt ist, wird das Bedienelement 3 an seinen linken Anschlag gezogen und der Endoskopkopf 5 maximal nach unten geschwenkt. Schiebt er ihn so weit wie möglich vom Griffabschnitt 12 weg, wird das Bedienelement 3 an seinen rechten Anschlag geschoben und der Endoskopkopf 12 maximal nach oben geschwenkt. Bei eingeführtem Finger kann der Benutzer dementsprechend ganz einfach mit derselben Hand, mit der er das Endoskop 1 hält, durch Bewegung des Fingers über den Manipulator 14 das Bedienelement 3 kontrollieren und den Endoskopkopf 5 in eine gewünschte Position bringen.

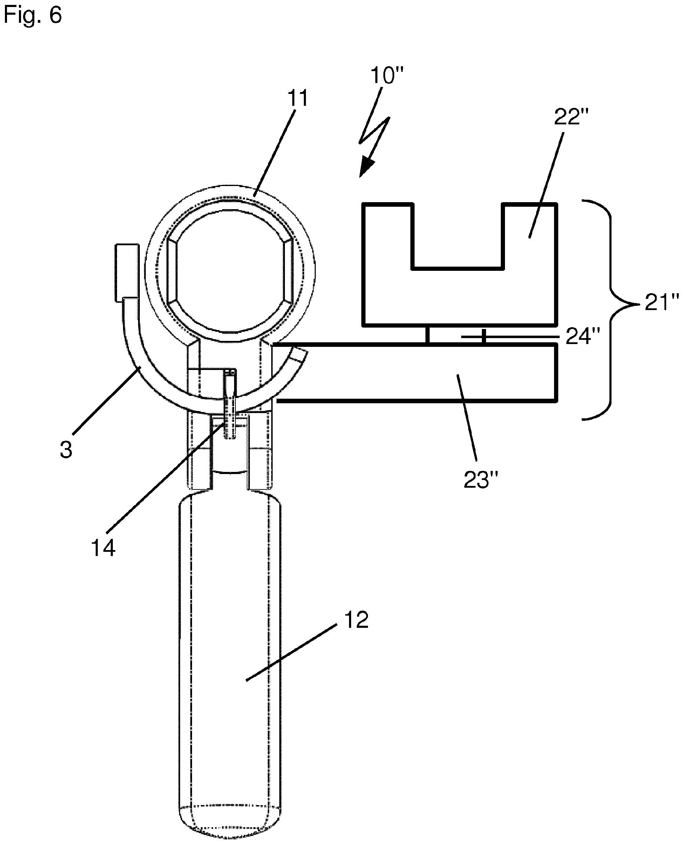

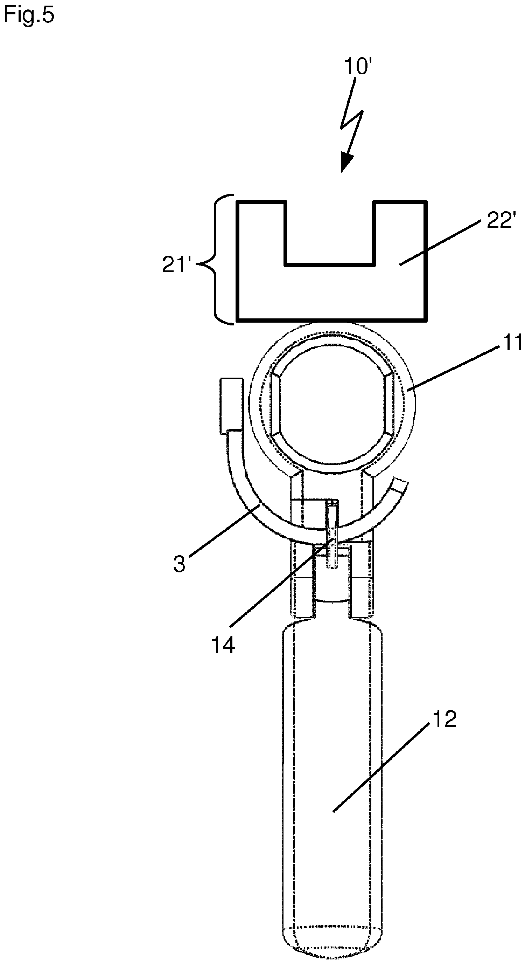

Die Figuren 5 und 6 zeigen jeweils Handgriffe 10', 10" die Varianten des Handgriffs 10 aus Figuren 1 und 2 darstellen und sich lediglich dadurch vom Handgriff 10 gemäß den Figuren 1 und 2 unterscheiden, dass der Handgriff zusätzlich einen Bildschirmaufnahmeabschnitt 21' bzw. 21" aufweist. Daher werden in den Figuren 5 und 6 für bereits im Zusammenhang mit den Figuren 1 und 2 beschriebenen Bestandteile der jeweiligen Variante des Handgriffs 10', 10" dieselben Bezugszeichen wie in den Figuren 1 und 2 verwendet und auf die korrespondierende Beschreibung verwiesen.

Die Bildschirmaufnahmeabschnitte 21',21" dienen jeweils dazu, einen nicht dargestellten Bildschirm am Handgriff 10', 10" anordnen zu können. Dieser Bildschirm kann insbesondere auch durch das Display eines Tablet-PCs oder eines Smartphones gebildet werden. Er ist im Regelfall fester kein Bestandteil des Handgriffs und/oder Endoskops 1 und wird zumindest in Datenkommunikation mit dem Endoskop 1 gebracht, um mit dem Endoskop 1 gewonnene Bildinformationen dem Benutzer unmittelbar anzeigen zu können. Falls diese Datenkommunikation mit dem Endoskop 1 durch eine kabelgebundene Verbindung zwischen Endoskop 1 und Bildschirm erfolgt, kann über diese kabelgebundene Verbindung auch das Endoskop 1 mit dem zu seinem Betrieb benötigten Strom versorgt werden, der dann durch die Stromversorgung des Bildschirms, insbesondere einen Akku, bereitgestellt werden kann.

Der in Figur 5 gezeigte Bildschirmaufnahmeabschnitt 21' besteht einfach aus einer Bildschirmhalterung 22', die schematisch einfach U-förmig dargestellt ist, wobei die Schenkel der U-förmigen Bildschirmhalterung 22' jeweils einen in der Perspektive der Figur 5 nicht sichtbaren Aufnahmeschlitz aufweisen, in den der Bildschirm eingesteckt und gegebenenfalls bevorzugt mit Fixiermitteln oder durch Klemmwirkung zwischen den Wandinnenflächen des Aufnahmeschlitzes fixiert werden kann.

Der Bildschirmaufnahmeabschnitt 21' ist an der dem Griffabschnitt 12 gegenüberliegenden Seite des Aufnahmeabschnitts 11 angeordnet und vorzugsweise lösbar mit dem Aufnahmeabschnitt 11 verbunden. Dies kann beispielsweise durch magnetische Wechselwirkung, insbesondere zwischen einem an der entsprechenden Stelle des Aufnahmeabschnitts 11 an oder in diesem angeordneten Magneten und einem am Bildschirmaufnahmeabschnitt 21' angeordneten Magneten, und/oder durch mechanische Wechselwirkung, insbesondere Aufstecken, Aufrasten oder Aufschrauben geschehen. Die Verbindung wird vorzugsweise erst dann hergestellt, wenn das Endoskop 1 bereits in den Aufnahmeabschnitt 11 des Handgriffs 10' eingeführt ist.

Der in Figur 6 gezeigte Bildschirmaufnahmeabschnitt 21" weist ebenfalls eine Bildschirmhalterung 22", die schematisch einfach U-förmig dargestellt auf. Auch die Schenkel der U-förmigen Bildschirmhalterung 22" weisen jeweils einen in der Perspektive der Figur 6 nicht sichtbaren Aufnahmeschlitz auf, in den der Bildschirm eingesteckt und gegebenenfalls bevorzugt mit Fixiermitteln oder durch Klemmwirkung zwischen den Wandinnenflächen des Aufnahmeschlitzes fixiert werden kann. Beim Bildschirmaufnahmeabschnitt 21" ist die Bildschirmhalterung 22" jedoch über ein Drehgelenk 24" mit einem Tragarm 22" verbunden.

Der Bildschirmaufnahmeabschnitt 21" ist in dem in Figur 6 gezeigten Ausführungsbeispiel seitlich an dem Aufnahmeabschnitt 11, beispielsweise an dessen Lagerabschnitt 16, angeordnet und wie der Bildschirmaufnahmeabschnitt 21' vorzugsweise lösbar mit dem Aufnahmeabschnitt 11 bzw. dem Lagerabschnitt 16 verbunden. Dies auch hier beispielsweise durch magnetische Wechselwirkung, insbesondere zwischen einem an der entsprechenden Stelle des Aufnahmeabschnitts 11 an oder in diesem angeordneten Magneten und einem am Bildschirmaufnahmeabschnitt 21' angeordneten Magneten, und/oder durch mechanische Wechselwirkung, insbesondere Aufstecken, Aufrasten oder Aufschrauben geschehen. Die Verbindung wird auch in diesem Fall vorzugsweise erst dann hergestellt, wenn das Endoskop 1 bereits in den Aufnahmeabschnitt 11 des Handgriffs 10" eingeführt ist.

### Bezugszeichenliste

- 1: Endoskop
- 2: Endoskopgehäuse
- 3: Bedienelement
- 4: Tubus
- 5: Endoskopkopf

- 10,10',10": Handgriff
- 11: Aufnahmeabschnitt

- 12: Griffabschnitt
- 12a: Verbindungsbereich
- 12b: Bohrung

- 13a,13b: Langloch

- 14: Manipulator
- 14a: erster Abschnitt
- 14b: Aufnahme
- 14c: zweiter Abschnitt
- 14d: Aufnahme
- 14e: Schlitz
- 14f: Bohrung

- 15: Öffnung

- 16: Lagerabschnitt
- 16a: erstes Teil
- 16b: zweites Teil

- 17a,17b: Fortsatz
- 18: Stift
- 19: Kanal
- 19a: erster Abschnitt
- 19b: zweiter Abschnitt

- 20: Bohrung

- 21',21": Bildschirmaufnahmeabschnitt
- 22',22": Halterung
- 23": Tragarm
- 24": Drehgelenk

- b: Breite
- D1: erste Drehachse
- D2: zweite Drehachse

## Patentansprüche

1. Handgriff (10,10',10") für ein Endoskop (1) , das ein Endoskopgehäuse (2) und einen Tubus (4) aufweist und wobei an dem Endoskopgehäuse (2) wenigstens ein Bedienelement (3) zur Veränderung einer Einstellung des Endoskops (1) vorhanden ist, wobei der Handgriff (10,10',10")
- einen Aufnahmeabschnitt (11) zur Aufnahme und Halterung des Endoskops (1) und
- einen mit dem Aufnahmeabschnitt (11) verbundenen Griffabschnitt (12) zum Halten des Endoskops (1) aufweist, wobei der Handgriff (10,10',10") ferner wenigstens einen Manipulator (14) zur Bedienung des Bedienelements (3) wenn der Handgriff (10,10',10") an dem Endoskop (1) angeordnet ist aufweist, wobei der Manipulator (14) relativ zu dem Aufnahmeabschnitt (11) und relativ zu dem Griffabschnitt (12) beweglich ist,
wobei der Manipulator (14) einen ersten Abschnitt (14a) und einen zweiten Abschnitt (14c) aufweist, wobei der erste Abschnitt (14a) relativ zum zweiten Abschnitt (14c) um eine erste Drehachse (D1) herum drehbar ist, **dadurch gekennzeichnet, dass** der zweite Abschnitt (14c) relativ zum Aufnahmeabschnitt (11) und/oder relativ zum Griffabschnitt (12) um eine zweite Drehachse (D2), die parallel zur ersten Drehachse (D1) verläuft, herum drehbar ist, sodass der Manipulator ein Bedienelement, das beim Bedienen auf einer Kreisbahn bewegt wird, bedienen kann.

2. Handgriff (10,10',10") nach Anspruch 1,
**dadurch gekennzeichnet, dass** der Aufnahmeabschnitt (11) relativ zum Griffabschnitt (12) abgewinkelt ist.

3. Handgriff (10,10',10") nach Anspruch 1 oder 2,
**dadurch gekennzeichnet, dass** der Handgriff (10,10',10") Mittel zur Veränderung der Position des Aufnahmeabschnitts (11) relativ zum Griffabschnitt (12) aufweist.

4. Handgriff (10,10',10") nach einem der Ansprüche 1 bis 3, **dadurch gekennzeichnet, dass** der Aufnahmeabschnitt (11) mindestens zweiteilig mit einem ersten Teil und einem zweiten Teil ausgeführt ist und dass der Griffabschnitt (12) und/oder der Manipulator (14) zwischen dem ersten Teil und dem zweiten Teil gehalten werden.

5. Handgriff (10,10',10") nach einem der Ansprüche 1 bis 3, **dadurch gekennzeichnet, dass** der Aufnahmeabschnitt (11) einen ein- oder mehrteiligen Lagerabschnitt 16 aufweist, an dem der Griffabschnitt (12) und/oder der Manipulator (14) gelagert sind.

6. Handgriff (10,10',10") nach einem der Ansprüche 1 bis 5, **dadurch gekennzeichnet, dass** der Manipulator (14) eine Aufnahme (14b) zum zumindest abschnittsweisen Einführen des Bedienelements (13) aufweist.

7. Handgriff (10,10',10") nach einem der Ansprüche 1 bis 6, **dadurch gekennzeichnet, dass** der Manipulator (14) eine Aufnahme (14d) zum zumindest abschnittsweisen Einführen eines Fingers eines Anwenders des Endoskops (1) aufweist.

8. Handgriff (10,10',10") nach einem der Ansprüche 1 bis 7, **dadurch gekennzeichnet, dass** der Manipulator (14) zumindest abschnittsweise in einem Innenraum des Handgriffs (10,10',10") verläuft.

9. Handgriff (10,10',10") nach einem der Ansprüche 1 bis 8, **dadurch gekennzeichnet, dass** der Manipulator (14) reversibel vom Handgriff (10,10',10") trennbar und wieder am Handgriff (10,10',10") befestigbar ist.

10. Handgriff (10,10',10") nach einem der Ansprüche 1 bis 9, **dadurch gekennzeichnet, dass** der Handgriff (10,10',10") einen Bildschirmaufnahmeabschnitt (21',21") zur Aufnahme eines Bildschirms aufweist.

11. Handgriff (10,10',10") nach Anspruch 10,
**dadurch gekennzeichnet, dass** der Bildschirmaufnahmeabschnitt (21',21") zur Aufnahme eines Bildschirms lösbar an dem Handgriff (10,10',10") angeordnet ist.

12. Endoskop (1) mit einem Endoskopgehäuse (2) und einem Tubus (4), bei dem an dem Endoskopgehäuse (2) wenigstens ein Bedienelement (3)zur Veränderung einer Einstellung des Endoskops (1) vorhanden ist,
**dadurch gekennzeichnet, dass** das Endoskop (1) einen Handgriff (10,10',10") nach einem der Ansprüche 1 bis 11 aufweist.

13. Verfahren zum Anordnen eines Handgriffs(10,10',10") an einem Endoskop (1) mit einem Endoskopgehäuse (2), an dem wenigstens ein Bedienelement (3) zur Veränderung einer Einstellung des Endoskops (1) vorhanden ist, und mit einem Tubus (4), wobei der Handgriff (10,10',10") einen Aufnahmeabschnitt (11) zur Aufnahme und Halterung des Endoskops (1), einen mit dem Aufnahmeabschnitt (11) verbundenen Griffabschnitt (12) zum Halten des Endoskops (1) und wenigstens einen Manipulator (14) zur Bedienung des Bedienelements (3) des Endoskops (1) aufweist, wobei der Manipulator (14) relativ zu dem Aufnahmeabschnitt (11) und relativ zu dem Griffabschnitt (12) beweglich ist, wobei das Verfahren die Schritte
- Aufnehmen des Endoskops (1) in einer Öffnung im Aufnahmeabschnitt (11) des Handgriffs (10,10',10"), und
- Verbinden des Bedienelements (3) mit dem Manipulator (14) aufweist,
wobei der Manipulator (14) während er mit dem Bedienelement (3) verbunden wird vom Handgriff (10,10',10") gelöst ist, nach dem Verbinden mit dem Bedienelement (3) wieder oder erstmalig am Handgriff (10,10',10") befestigt wird und dass ein Handgriff (10,10',10") verwendet wird, der einen Manipulator (14) hat, welcher einen ersten Abschnitt (14a) und einen zweiten Abschnitt (14c) aufweist, wobei der erste Abschnitt (14a) relativ zum zweiten Abschnitt (14c) um eine erste Drehachse (D1) herum drehbar ist und **dadurch gekennzeichnet, dass** der zweite Abschnitt (14c) relativ zum Aufnahmeabschnitt (11) und/oder relativ zum Griffabschnitt (12) um eine zweite Drehachse, (D2) die parallel zur ersten Drehachse (D1) verläuft, herum drehbar ist, sodass der Manipulator ein Bedienelement, das beim Bedienen auf einer Kreisbahn bewegt wird, bedienen kann und,
dass das Bedienelement (3) mit dem Manipulator (14) verbunden wird, indem das Bedienelement (3) in eine am ersten Abschnitt (14a) des Manipulators (14) vorgesehene Aufnahmeöffnung (14b) eingeführt wird,
dass der so mit dem Bedienelement (3) verbundene Manipulator (14) in einen Kanal (19) im Aufnahmeabschnitt (11) eingelegt und über ein Bauelement, das die zweite Drehachse (D2) mit dem Aufnahmeabschnitt (11) verbunden wird.

14. Verfahren nach Anspruch 13,
**dadurch gekennzeichnet, dass** der Kanal nach dem Einlegen des mit dem Bedienelement (3) verbundenen Manipulators (14) und nach dem Verbinden über die zweite Drehachse (D2) mit dem Aufnahmeabschnitt (11) mit einem Deckel verschlossen wird.

15. Verfahren nach einem der Ansprüche 13 oder 14,
**dadurch gekennzeichnet, dass** ein Handgriff (10,10',10") verwendet wird, der einen Bildschirmaufnahmeabschnitt (21',21") hat und dass ein Bildschirm in dem Bildschirmaufnahmeabschnitt (21',21") angeordnet und zumindest eine Signalkommunikation mit dem Endoskop (1) hergestellt wird.

16. Verfahren nach einem der Ansprüche 13 bis 15,
**dadurch gekennzeichnet, dass** ein Handgriff (10,10',10") verwendet wird, der einen lösbaren Bildschirmaufnahmeabschnitt (21',21") hat und dass der lösbare Bildschirmaufnahmeabschnitt (21',21‴) erst nach dem Aufnehmen des Endoskops (1) in einer Öffnung im Aufnahmeabschnitt (11) des Handgriffs (10,10',10") mit dem Handgriff (10,10',10") verbunden wird.

## Claims

1. Handle (10, 10', 10") for an endoscope (1), which comprises an endoscope housing (2) and a tube (4), and wherein at least one control element (3) for changing an adjustment of the endoscope (1) is provided on the endoscope housing (2), wherein the handle (10, 10', 10") comprises
- a receiving section (11) for receiving and retaining the endoscope (1) and
- a handle section (12), which is connected to the receiving section (11), for holding the endoscope (1), wherein the handle (10, 10', 10") further comprises at least one manipulator (14) for controlling the control element (3) when the handle (10, 10', 10") is disposed on the endoscope (1), wherein the manipulator (14) is movable relative to the receiving section (11) and relative to the handle section (12), wherein the manipulator (14) comprises a first section (14a) and a second section (14c), wherein the first section (14a) is rotatable relative to the second section (14c) around a first axis of rotation (D1),
**characterized in that** the second section (14c) is rotatable relative to the receiving section (11) and/or relative to the handle section (12) around a second axis of rotation (D2) which extends parallel to the first axis of rotation (D1) such that the manipulator can control a control element which, when being controlled, is moved along a circular path.

2. Handle (10, 10', 10") in accordance with claim 1,
**characterized in that** the receiving section (11) is angled relative to the handle section (12).

3. Handle (10, 10', 10") in accordance with claim 1 or 2,
**characterized in that** the handle (10, 10', 10") comprises means for changing the position of the receiving section (11) relative to the handle section (12).

4. Handle (10, 10', 10") in accordance with any of claims 1 to 3,
**characterized in that** the receiving section (11) is implemented in at least two parts having a first part and a second part, and **in that** the handle section (12) and/or the manipulator (14) is held between the first part and the second part.

5. Handle (10, 10', 10") in accordance with any of claims 1 to 3,
**characterized in that** the receiving section (11) comprises a one or two piece bearing section (16) on which the handle section (12) and/or the manipulator (14) is(are) mounted.

6. Handle (10, 10', 10") in accordance with any of claims 1 to 5,
**characterized in that** the manipulator (14) comprises an opening (14b) for the at least partial insertion of the control element (13).

7. Handle (10, 10', 10") in accordance with any of claims 1 to 6,
**characterized in that** the manipulator (14) comprises an opening (14d) for the at least partial insertion of a finger of a user of the endoscope (1).

8. Handle (10, 10', 10") in accordance with any of claims 1 to 7,
**characterized in that** the manipulator (14) extends at least partially into an inner chamber of the handle (10, 10', 10").

9. Handle (10, 10', 10") in accordance with any of claims 1 to 8,
**characterized in that** the manipulator (14) is reversibly removable from the handle (10, 10', 10") and can be reattached to the handle (10, 10', 10").

10. Handle (10, 10', 10") in accordance with any of claims 1 to 9,
**characterized in that** the handle (10, 10', 10") comprises a monitor receiving section (21', 21") for receiving a monitor.

11. Handle (10, 10', 10") in accordance with claim 10,
**characterized in that** the monitor receiving section (21', 21") for receiving a monitor is detachably arranged on the handle (10, 10', 10").

12. Endoscope (1) having an endoscope housing (2) and a tube (4), and on which at least one control element (3) for changing an adjustment of the endoscope (1) is provided, **characterized in that** the endoscope (1) comprises a handle (10, 10', 10") in accordance with any of claims 1 to 11.

13. Method for arranging a handle (10, 10', 10") on an endoscope (1) having an endoscope housing (2) on which at least one control element (3) for changing an adjustment of the endoscope is provided, and with a tube (4), wherein the handle 10, 10', 10") comprises a receiving section (11) for receiving and retaining the endoscope (1), a handle section (12), which is connected to the receiving section (11), for holding the endoscope (1) and at least one manipulator (14) for controlling the control element (3), wherein the manipulator (14) is movable relative to the receiving section (11) and relative to the handle section (12), wherein the method comprises the steps
- inserting the endoscope (1) into an opening in the receiving section (11) of the handle (10,m 10', 10"), and
- connecting the control element (3) to the manipulator (14), wherein the manipulator, while being attached to the control element (3), (14) is detached from the handle (10, 10', 10"), after being attached to the control element (3), is attached to the handle (10, 10', 10") again or for the first time and wherein a handle (10, 10', 10") is used which has a manipulator (14) which comprises a first section (14a) and a second section (14c), wherein the first section (14a) is rotatable relative to the second section (14c) around a first axis of rotation (D1),
**characterized in that** the second section (14c) is rotatable relative to the receiving section (11) and/or relative to the handle section (12) around a second axis of rotation (D2) which extends parallel to the first axis of rotation (D1) such that the manipulator can control a control element (3) which, while being controlled, moves along a circular path and **in that** the control element (3) is connected to the manipulator (14) by inserting the control element (3) into an opening (14b) provided in the first section (14a) such that the manipulator, having been thus connected to the control element (3), is inserted into a channel (19) in the receiving section (11) and is connected via a component forming the second axis of rotation (D2) to the receiving section (11).

14. Method in accordance with claim 13,
**characterized in that** the channel, after the manipulator (14) connected to the control element (3) has been inserted and has been connected, via the second axis of rotation (D2), to the receiving section (11), is closed with a cover.

15. Method in accordance with either of claims 13 or 14,
**characterized in that** a handle (10, 10', 10") is used which has a detachable monitor receiving section (21', 21") and **in that** a monitor is arranged in the monitor receiving section (21', 21") and **in that** at least one signal communication with the endoscope (1) is established.

16. Method in accordance with any of claims 13 to 15,
**characterized in that** a handle (10, 10', 10") is used which has a detachable monitor receiving section (21', 21") and **in that** the detachable receiving section (21', 21") is not connected to the handle (10, 10', 10") until after the endoscope (1) has been inserted into an opening in the receiving section (11) of the handle (10, 10', 10").

## Revendications

1. Poignée (10, 10', 10") pour un endoscope (1) ayant un boîtier d'endoscope (2) et un tube (4), et
le boîtier d'endoscope (2) ayant au moins un élément de manœuvre (3) pour modifier un réglage de l'endoscope (1),
la poignée (10, 10', 10") ayant
- un segment récepteur (11) pour recevoir et fixer l'endoscope (1), et
- un segment de poignée (12) relié au segment récepteur (11) pour tenir l'endoscope (1),
- la poignée (10, 10', 10") ayant en outre au moins un manipulateur (14) pour actionner l'élément de manœuvre (3) lorsque la poignée (10, 10', 10") est installée sur l'endoscope (1),
* le manipulateur (14) étant mobile par rapport au segment récepteur (11) et par rapport au segment de poignée (12),
- le manipulateur (14) ayant un premier segment (14a) et un second segment (14c),
* le premier segment (14a) pouvant tourner par rapport au second segment (14c) selon un premier axe de rotation (D1),
poignée **caractérisée en ce que**
le second segment (14c) peut tourner par rapport au segment récepteur (11) et/ou par rapport au segment de poignée (12) autour d'un second axe de rotation (D2) parallèle au premier axe de rotation (D1) de façon que le manipulateur puisse actionner un élément de manœuvre qui par l'actionnement se déplace sur une trajectoire circulaire.

2. Poignée (10, 10', 10") selon la revendication 1,
**caractérisée en ce que**
le segment récepteur (11) est coudé par rapport au segment de poignée (12).

3. Poignée (10, 10', 10") selon la revendication 1 ou 2,
**caractérisée en ce que**
la poignée (10, 10', 10") comporte des moyens pour modifier la position du segment récepteur (11) par rapport au segment de poignée (12).

4. Poignée (10, 10', 10") selon l'une des revendications 1 à 3, **caractérisée en ce que**
le segment récepteur (11) est réalisé au moins en deux parties avec une première partie et une seconde partie et le segment de poignée (12) et/ou le manipulateur (14) sont tenus entre la première partie et la seconde partie.

5. Poignée (10, 10', 10") selon l'une des revendications 1 à 3, **caractérisée en ce que**
le segment récepteur (11) a un segment de palier (16) en une ou plusieurs parties recevant le segment de poignée (12) et/ou le manipulateur (14).

6. Poignée (10, 10', 10") selon l'une des revendications 1 à 5, **caractérisée en ce que**
le manipulateur (14) a un logement (14b) pour introduire au moins par segment, l'élément de manœuvre (13).

7. Poignée (10, 10', 10") selon l'une des revendications 1 à 6, **caractérisée en ce que**
le manipulateur (14) a un logement (14d) pour introduire au moins par segment, un doigt de l'utilisateur de l'endoscope (1).

8. Poignée (10, 10', 10") selon l'une des revendications 1 à 7, **caractérisée en ce que**
le manipulateur (14) passe au moins par segment dans le volume intérieur de la poignée (10, 10', 10").

9. Poignée (10, 10', 10") selon l'une des revendications 1 à 8, **caractérisée en ce que**
le manipulateur (14) est relié de manière réversible à la poignée (10, 10', 10") pour pouvoir en être séparé et être de nouveau fixé à la poignée (10, 10', 10").

10. Poignée (10, 10', 10") selon l'une des revendications 1 à 9, **caractérisée en ce que**
la poignée (10, 10', 10") a un segment de support d'écran (21', 21") pour recevoir un écran.

11. Poignée (10, 10', 10") selon la revendication 10,
**caractérisée en ce que**
le segment support d'écran (21', 21") est installé de manière amovible sur la poignée (10, 10', 10") pour recevoir un écran.

12. Endoscope (1) comportant un boîtier d'endoscope (2) et un tube (4) le boîtier d'endoscope (2) comportant au moins un élément de manœuvre (3) pour modifier le réglage de l'endoscope (1),
endoscope (1) **caractérisé en ce que**
il comporte une poignée (10, 10', 10") selon l'une des revendications 1 à 11.

13. Procédé de montage d'une poignée (10, 10', 10") sur un endoscope (1) ayant un boîtier d'endoscope (2) muni d'au moins un élément de manœuvre (3) pour modifier un réglage de l'endoscope (1) et un tube (4),
- la poignée (10, 10', 10") ayant un segment récepteur (11) pour recevoir et fixer l'endoscope (1), un segment de poignée (12) relié au segment récepteur (11) pour tenir l'endoscope (1) et au moins un manipulateur (14) pour actionner l'élément de manœuvre (3) de l'endoscope (1),
- le manipulateur (14) étant mobile par rapport au segment récepteur (11) et par rapport au segment de poignée (12),
- le procédé comprenant les étapes suivantes :
- recevoir l'endoscope (1) dans une ouverture du segment récepteur (11) de la poignée (10, 10', 10"), et
- relier l'élément de manœuvre (3) au manipulateur (14),
selon lequel
pendant qu'on le relie à l'élément de manœuvre (3), le manipulateur (14) est séparé de la poignée (10, 10', 10"), après la liaison avec l'élément de manœuvre (3) la poignée est refixée ou est fixée la première fois à la poignée (10, 10', 10"), et
on utilise une poignée (10, 10', 10") ayant un manipulateur (14) qui a un premier segment (14a) et un second segment (14c),
* le premier segment (14a) pouvant tourner par rapport au second segment (14c) autour d'un premier axe de rotation (D1),
**caractérisé en ce que**
- le second segment (14c) peut tourner par rapport au segment récepteur (11) et/ou par rapport au segment de poignée (12) autour d'un second axe de rotation (D2) parallèle au premier axe de rotation (D1) de façon que le manipulateur puisse activer un élément de manœuvre qui est déplacé par l'actionnement sur une trajectoire circulaire, et
- l'élément de manœuvre (3) est relié au manipulateur (14) par l'engagement de l'élément de manœuvre (3) dans un premier segment (14a) de l'ouverture de réception (14b) du manipulateur (14),
- le manipulateur (14) ainsi relié à l'élément de manœuvre (3) se place dans un canal (19) du segment récepteur (11) et est relié par un élément de construction reliant le second axe de rotation (D2) au segment récepteur (11).

14. Procédé selon la revendication 13,
**caractérisé en ce que**
après la mise en place du manipulateur (14) relié à l'élément de manœuvre (3) et après l'avoir relié par le second axe de rotation (D2) au segment récepteur (11), on ferme le canal avec un couvercle.

15. Procédé selon l'une des revendications 13 ou 14,
**caractérisé en ce que**
on utilise une poignée (10, 10', 10") ayant un segment de support d'écran (21', 21") et un écran est installé dans le segment de support d'écran (21', 21") et on établit au moins une communication de signal avec l'endoscope (1).

16. Procédé selon l'une des revendications 13 à 15,
**caractérisée en ce que**
on utilise une poignée (10, 10', 10") qui a un segment de support d'écran (21', 21") amovible et ce segment de support d'écran (21', 21") amovible est relié à la poignée (10, 10', 10") seulement après avoir logé l'endoscope (1) dans une ouverture du segment récepteur (11) de la poignée (10, 10', 10").
